# EUROPÄISCHE PATENTSCHRIFT

(11) **EP 1 496 974 B1**
(45) Veröffentlichungstag und Bekanntmachung des Hinweises auf die Patenterteilung: **25.02.2009**
(21) Anmeldenummer: 03746835.2
(22) Anmeldetag: 22.04.2003
(51) Int. Cl.: A61M 25/01, A61M 25/00, A61F 2/06

(54) **MEDIZINISCHER RETRIEVER**
MEDICAL RETRIEVER
EXTRACTEUR MEDICAL

(30) Priorität: 20.04.2002 DE 10217757
(43) Veröffentlichungstag der Anmeldung: 19.01.2005
(73) Patentinhaber: Brassel, Friedhelm, 47055 Duisburg (DE)
(72) Erfinder: Brassel, Friedhelm, 47055 Duisburg (DE)
(74) Vertreter: Schneiders, Josef
(86) Internationale Anmeldenummer: PCT/EP2003/004164
(87) Internationale Veröffentlichungsnummer: WO 2003/089039

(56) Entgegenhaltungen:
- WO-A-91/13592
- US-A- 4 994 069
- US-A- 5 195 954
- US-A- 5 868 754
- US-B1- 6 251 128
- US-B1- 6 270 495

## Beschreibung

Die Erfindung betrifft eine Vorrichtung zum Extrahieren einer Spiralwendel aus einem Blutgefäß. Die Erfindung betrifft weiterhin die Kombination einer solchen Vorrichtung mit einem Katheter.

Zur Behandlung von Gefäßdefekten, etwa Aneurysmen oder arteriellen Fisteln, werden vielfach Embolisierungsspiralen eingesetzt, die mit Mikrokathetern am Einsatzort plaziert werden. Sogenannte "floating coils" werden dabei dicht am Einsatzort freigesetzt und treiben im Blutstrom zum Zielpunkt. Andere Spiralformen werden am Zielpunkt vom Katheter gelöst. Die Plazierungstechniken sind sehr zuverlässig, jedoch besteht immer wieder die Notwendigkeit, eine fehlplazierte oder abgeschwemmte Spirale wieder zu extrahieren. Bekannte Extraktionsvorrichtungen versagen aber in Gefäßen, die so eng sind, daß Sie ein "Greifen" der steckengebliebenen Spirale mit herkömmlichen Greifvorrichtungen nicht mehr erlauben.

Eine Vorrichtung zur Extrahierung einer im Herzen eingewachsenen Herzschrittmacherelektrode mit einem als Spiralwendel ausgebildeten Elektrodenkabels ist aus der internationalen Patentanmeldung WO 91/13592 A1 bekannt und verfügt über ein Verankerungsteil in Gestalt eines geschlitzten Rohrsegmentes, das am distalen Ende des Zugdrahtes befestigt ist und dessen zum Rohr weisende Enden nach außen leicht vorgespreizt sind. Durch das Ziehen am Zugdraht wird das vorgespreizte, geschlitzte Rohrsegment aktiviert, wobei die Rohrsegmenthälften auseinandergespreizt werden und mit ihren freien Enden ein Eingriff in den Innendurchmesser der Spiralwendel des Elektrodenkabels erfolgen kann.

Bei der bekannten Vorrichtung ist es erforderlich, je nach dem Innendurchmesser des Elektrodenkabels unterschiedliche Größen für das Verankerungsteil der Extraktionsvorrichtung vorzusehen. Ein weiterer Nachteil besteht darin, daß die Rohrsegmenthälften bei einem zu großen Innendurchmesser umknicken können und dann eine sichere Verankerung nicht mehr gewährleistet ist. Außerdem arbeitet diese bekannte Extraktionsvorrichtung nur bei mehrfach gewendelten Elektrodenkabeln zuverlässig, insbesondere vierfach gewendelten Elektroden. Eine Anwendung zur Extraktion verlorengegangener Coils, die zumeist noch in sich gewendelt und verknäult sind, ist nicht denkbar.

US-A-5 868 754 zeigt eine Vorrichtung, die zur Extraktion einer Spiralwendel aus einem Blutgefäß geeignet ist und einen Führungsdraht, der in seinem distalen Bereich unter Ausbildung einer Schlaufenstruktur auf sich selbst zurückgebogen ist, aufweist. Die Vorrichtung ist mit Fasern versehen, die sich um die zu extrahierende Spiralwendel wickeln und diese festhalten.

US-A-4 994 069 beschreibt einen Retriever mit einem gewundenen distalen Ende, der zur Extraktion von Spiralwendeln bestimmt ist. Die gewundene Struktur weist nach distal und ist dazu bestimmt, in eine verknäuelte Spiralwendel hineingedreht zu werden und diese festzuhalten.

Spiralwendel, die sich bei Längen von mehreren Zentimetern regelmäßig verknäulen, können, je nach Art ihres Materials, in den Gefäßen zu lebensgefährlichen Verschlüssen führen. Bei Abschwemmung, insbesondere auch in Herz, Gehirn oder Lunge, drohen lebensgefährliche Komplikationen.

Ausgehend von diesem Stand der Technik besteht die Aufgabe der Erfindung darin, eine Extraktionsvorrichtung zur Verfügung zu stellen, die Spiralwendeln unterschiedlichster Ausgestaltung, die einfach oder mehrfach in sich gewendelt sind, extrahieren kann. Insbesondere soll es die Erfindung ermöglichen, verlorengegangene Spiralen auch aus englumigen Gefäßen zu extrahieren.

Diese Aufgabe wird mit einer Vorrichtung zum Extrahieren einer Spiralwendel aus einem Blutgefäß gelöst gemäß Anspruch 1.

Die erfindungsgemäße Vorrichtung besteht im Grunde genommen aus einem herkömmlichen Führungsdraht, dessen distales Ende mechanisch auf sich selbst zurückgebogen ist, wobei sich eine hakenförmige Struktur ausbildet. Das zurückgebogene distale Ende macht in der Regel eine Länge von 1 bis 3 cm +aus. Die zum Einsatz kommenden Führungsdrähte sind solche, wie sie in der Mikrokathetertechnik eingesetzt werden, d. h. mit einem Durchmesser von 0,1 bis 0,5 mm. Zur Verbesserung der Funktionsfähigkeit sind derartige Führungsdrähte zweckmäßigerweise mit einer üblichen hydrophilen Beschichtung versehen.

Es ist essentiell, daß die erfindungsgemäß verwandten Führungsdrähte in ihrem funktionellen Teil hoch flexibel ausgebildet sind, daß sie sich den Windungen des Gefäßsystems im Körper ohne weiteres anpassen können und dessen Verlauf folgen können. Die Flexibilität ist für den Einfang- und Verhakungsprozeß beim Extrahieren einer verlorengegangenen Spiralwendel förderlich. Führungsdrähte, deren Flexibilität distal zunimmt, sind besonders zweckmäßig. Eine solche Ausbildung ermöglicht es, daß sich das freie Ende des Führungsdrahtes mit der eingefangenen Spirale um einen proximal gelegenen Teil des Führundsdrahtes wickeln kann.

Erfindungsgemäß weist der Verankerungsteil eine hakenförmige Struktur auf, d. h. das distale Ende des Führungsdrahts ist auf sich selbst zurückgebogen und bildet am distalen Ende eine bogenförmige Struktur (Schlaufe) aus, wobei sich das zurückgebogene (und nach proximal weisende) Ende des Führungsdrahts frei bewegen kann. Das Ende ist korkenzieherartig gewunden.

Erfindungsgemäß verläuft die durch das Zurückbiegen des distalen Führungsdrahtendes entstandene Schlaufe nicht in Richtung des Führungsdrahts sondern ist zumindest im distalen Bereich in ihrer Ebene gegen den Verlauf des Führungsdrahtes leicht abgewinkelt. Diese Ausführungsform läßt sich besonders leicht durch Gefäßbiegungen und Verästelungen steuern. Vorzugsweise ist auch das distale Ende des Führungsdrahtes (das freie Ende der Schlaufe) gegen den Verlauf des Führungsdrahtes abgewinkelt, was für den Einfangvorgang förderlich ist.

Das freie Ende des Führungsdrahts ist in sich korkenzieherartig gewunden, was eine bessere Verhakung mit zu extrahierenden Spiralwendeln ermöglicht. Gemäß einer weiteren zweckmäßigen Ausführungsform kann das zurückgebogene Ende des Führungsdrahts an seiner Spitze eine kugelförmige Verdickung aufweisen, die ebenfalls den Verhakungsvorgang fördert.

Zur Sicherung der Hakenstruktur kann es zweckmäßig sein, das zurückgebogene freie Ende des Führungsdrahts mit einer Manschette am Führungsdraht selbst festzulegen. In diesem Fall wird die vom Führungsdraht an seinem distalen Ende ausgebildete schlaufenförmige Struktur von der Manschette gesichert. Die Manschette kann aus einem beliebigen körperverträglichen Material gebildet sein, ist aber zweckmäßigerweise aus einem röntgendichten Metall. Gemäß einer weiteren bevorzugten Ausführungsform kann das freie Ende der schlaufenförmigen Struktur aus der Manschette freigesetzt werden.

Zur Kontrolle des Extraktionsvorgangs ist es besonders zweckmäßig, wenn auch das freie Ende des Führungsdrahts zumindest teilweise röntgendicht ausgebildet ist. Dies kann dadurch geschehen, daß für den Führungsdraht selbst in diesem Bereich ein röntgendichtes Metall verwandt wird, beispielsweise Platin oder Gold, oder auch durch die Verwendung einer röntgendichten Kunststoffbeschichtung, beispielsweise durch Einlagerung von röntgendichtem Metallstaub in die Kunststoffbeschichtung. Solche Maßnahmen sind dem zuständigen Durchschnittsfachmann hinlänglich bekannt.

Mit der erfindungsgemäßen Vorrichtung können im Gefäßsystem verlorengegangene oder deplazierte Spiralwendeln auch aus engen Gefäßen extrahiert werden. Dazu wird die Spiralwendel mit dem Verankerungsteil des erfindungsgemäßen Retrievers hinterfahren und durch Zurückziehen des Führungsdrahts eingefangen. Durch Drehung des Mikroführungsdrahts wird die Metallspirale dann um das Zentrum (die Achse) des Führungsdrahts gewickelt, wobei sich das distale hochflexible Ende des umgeknickten Bereichs um die Drehachse des Führungsdrahts bzw. die eingefangene Spirale wickelt.

Beim Zurückziehen muß die einmal festgelegte Drehrichtung des Drahtes beibehalten werden, da sich ansonsten der Verschlußfaden wieder abwickelt - der Haken öffnet sich - und die Spirale freigibt.

Die Röntgenmarkierung erlaubt die vollständige Überwachung des Einfang- bzw. Extraktionsprozesses.

Die Erfindung betrifft überdies die Kombination der erfindungsgemäßen Vorrichtung mit einem Katheter, insbesondere einem Mikrokatheter. Gemäß einer bevorzugten Ausführungsform dieser Kombination weist die erfindungsgemäße Vorrichtung eine übliche Betätigungseinrichtung auf, bei der der Führungsdraht drehfest mit einer Griffhülse verbunden und an einer Buchse angebracht ist, die im oder auf dem proximalen Katheterende befestigt ist.

Die Erfindung wird durch die beigefügten Abbildungen näher erläutert. Es zeigen in vergrößerter Darstellung:

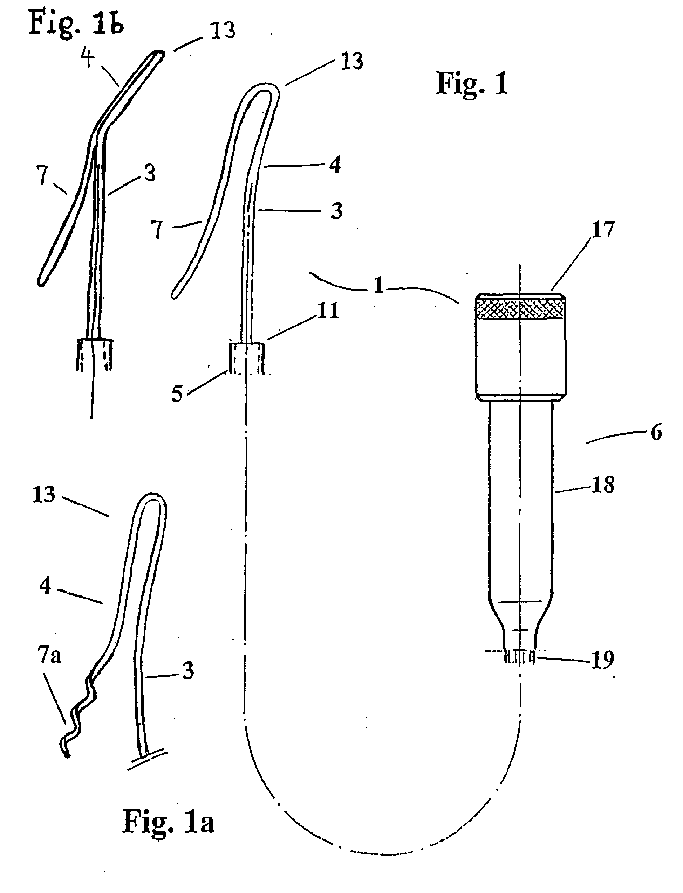

- Figur 1: ein Beispiel für eine Kombination eines Katheters mit einer Vorrichtung zur Extraktion von Spiralwendeln;
- Figur 1 a: eine Ausführungsform der vorliegenden Erfindung mit korkenzieherförmig gewundenem freien Ende des Führungsdrahts;
- Figur 1b: das Beispiel von Fig. 1 in seitlicher Ansicht;
- Figur 2: einen Ausschnitt aus einer herkömmlichen Spiralwendel und
- Figur 3: eine von dem erfindungsgemäßen Retriever eingefangene Spiralwendel.

Die in Figur 1 dargestellte Retriever-Kombination 1 besteht aus dem zum Verankerungsteil 2 umgebogenen Führungsdraht 3, dem Katheter 5 sowie der Betätigungsvorrichtung 6. Die Darstellung dient lediglich der Veranschaulichung und ist nicht maßstabsgerecht. Hinsichtlich des windungsfacien Ausgestaltung des freien Endes 7 des Führungsdrahst entspricht sie nicht des Erfindung. Der Führungsdraht 3 ist durch das Katheterrohr 5 geführt und in der Betätigungsvorrichtung 6 an der Griffhülse 17 festgelegt und kann durch drehen der Griffhülse 17 gegen die Buchse 18 gedreht werden.

An der der Griffhülse 17 gegenüberliegenden Seite der Buchse 18 ist das proximale Ende 19 des Katheterrohres 5 gezeigt.

Der Führungsdraht 3 ist zur Ausbildung des Verankerungsteils an seinem distalen Ende auf sich selbst zurückgebogen, wobei das freie Ende 7 in Richtung auf das distale Ende 11 des Katheters 5 weist. Die dadurch gebildete Schlaufenstruktur 4 hat ein distales Ende 13, mit dem der Retriever durch das jeweilige Blutgefäß vorgeschoben wird. Die Schlaufe 4 des Verankerungsteils 2 ist an ihrem distalen Ende leicht gegen die Achse des Führungsdrahtkörpers geneigt und ermöglicht so eine besonders leichte Manövrierung des Retrievers 1 durch Biegungen und Verzweigungen des Blutgefäßsystems.

Figur 1a zeigt eine erfindungsgemäße Ausführungsform, bei der das freie Ende des Führungsdrahts 3 jenseits der Schlaufe 4 zu einer Korkenzieherstruktur 7a umgeformt ist. Besonders deutlich ist hier die Schlaufenform 4, die - übertrieben dargestellt - das Verankerungsteil an seinem distalen Ende stumpf macht und damit Verletzungen im Gefäßsystem vermeidet. Im übrigen ist der Führungsdraht 3 selbst aus einem für Mikroführungsdrähte üblichen hochelastischen Material mit hydrophiler Beschichtung ausgebildet, das selbst wenig verletzungsträchtig ist.

Figur 1b zeigt die Schlaufe 4 aus Fig. 1 in seitlicher Ansicht. Die Neigung der Schlaufe gegen die Achse/den Verlauf des Führungsdrahtes 3 wird deutlich, ebenso der abgewinkeite Verlauf des freien Endes 7, das wegendes windungs-freien Ausführung des freien Endes 7 nicht der Erfindung entspricht.

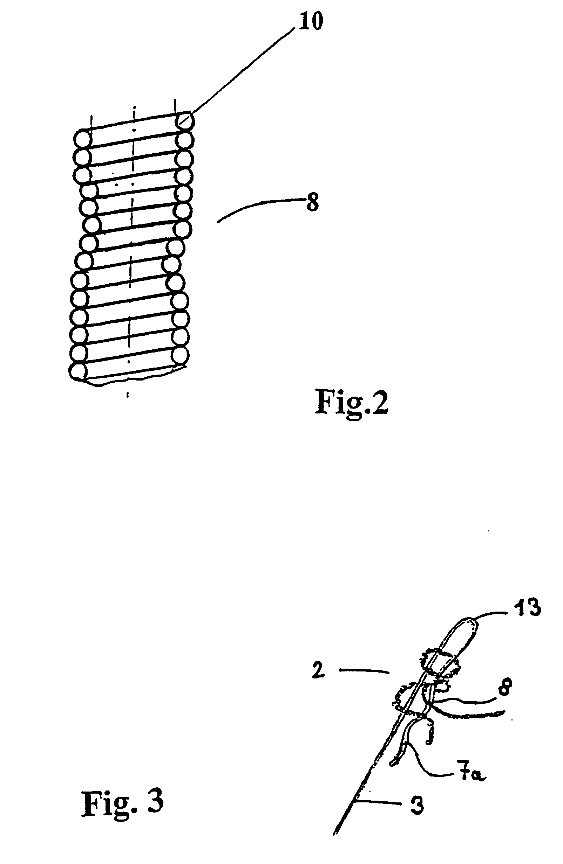

Figur 2 zeigt einen Abschnitt einer üblichen Embolisierungsspirale 8 im Schnitt mit einzelnen Wendeln 10. Diese Embolisierungsspiralen 8 haben bei einem effektiven Durchmesser von beispielsweise 0,1 bis 0,5 mm häufig eine Länge von mehreren cm und neigen daher zu einer inneren Verknäulung. Knäuel solcher Spiralwendeln erlauben das Einhaken des erfindungsgemäßen Verankerungsteils, das Abwickeln des Knäuels und das Aufwickeln der dadurch gefangenen Spirale um den Führungsdraht 3 im Bereich es Verankerungsteils 2 durch einfaches drehen. Das Einfangen geschieht dabei in erster Linie über das freie Ende 7, das als Haken wirkt; durch das Aufwickeln der Spirale schließt sich dieser Haken und bleibt die Spiralwendel gefangen.

Figur 3 zeigt ausschnittsweise eine mit dem erfindungsgemäßen Verankerungsteil 2 eingefangene Spiralwendel 8, die sich im Verankerungsteil verfangen hat und durch Drehen des Führungsdrahts 3 um das Verankerungsteil 2 gewickelt hat. Das Verankerungsteil 2 weist in diesem Beispiel keine Sicherungsmanschette auf, was einer besonders atraumatischen Ausbildung entspricht und insbesondere für das Einfangen von Coils aus sehr englumigen Gefäßästen zweckmäßig ist.

## Patentansprüche

1. Vorrichtung zum Extrahieren einer Spiralwendel aus einem Blutgefäß, mit einem Führungsdraht (3), der in seinem distalen Bereich unter Ausbildung einer Schlaufenstruktur (13) auf sich selbst zurückgebogen ist, so dass sich ein Verankerungsteil (2) ausbildet, wobei das freie Ende (7) des Führungsdrahtes (3) in Richtung auf das proximale Ende des Führungsdrahtes (3) weist und mit der zur extrahierenden Spiralwendel (8) verhakbar ist, **dadurch gekennzeichnet, daß** das freie Ende (7) des Führungsdrahtes (3) korkenzieherartig gewunden ist und das distale Ende der Schlaufenstruktur (13) gegenüber der Achse des Führungsdrahtkörpers abgewinkelt ist.

2. Vorrichtung nach Anspruch 1, **dadurch gekennzeichnet, daß** der Führungsdraht (3) eine hydrophile Beschichtung aufweist.

3. Vorrichtung nach einem der Ansprüche 1 oder 2, **dadurch gekennzeichnet, daß** das Verankerungsteil (2) als Haken ausgebildet ist.

4. Vorrichtung nach einem der vorstehenden Ansprüche, **dadurch gekennzeichnet, daß** der Führungsdraht (3) an seinem freien Ende (7) einen Durchmesser von 0,1 bis 0,5 mm aufweist.

5. Vorrichtung nach einem der vorstehenden Ansprüche, **dadurch gekennzeichnet, daß** das freie Ende (7) mit einer Manschette (4) unter Ausbildung einer Schlaufe (13) am Führungsdraht (3) gesichert ist.

6. Vorrichtung nach Anspruch 5, **dadurch gekennzeichnet, daß** die Manschette (4) röntgendicht ausgebildet ist.

7. Vorrichtung nach einem der vorstehenden Ansprüche, **dadurch gekennzeichnet, daß** das freie Ende (7) zumindest teilweise röntgendicht ausgebildet ist.

8. Vorrichtung nach Anspruch 7, **dadurch gekennzeichnet, daß** das freie Ende (7) des Führungsdrahts (3) eine röntgendichte Beschichtung aufweist.

9. Vorrichtung nach Anspruch 8, **dadurch gekennzeichnet, daß** die röntgendichte Beschichtung eine Metallstaubeinlagerung aufweist.

10. Vorrichtung nach einem der vorstehenden Ansprüche, **dadurch gekennzeichnet, daß** das freie Ende (7) des Führungsdrahts (3) hochflexibel ausgebildet ist, so daß sie sich bei Rotation des Führungsdrahts (3) um den Führungsdraht (3) wickelt.

11. Kombination einer Vorrichtung gemäß einem der vorstehenden Ansprüche 1 bis 10 mit einem Katheter, insbesondere einem Mikrokatheter.

12. Kombination gemäß Anspruch 11, **gekennzeichnet durch** eine Betätigungseinrichtung (6), an der der Führungsdraht (3) drehfest mit einer Griffhülse (17) verbunden ist und an einer Buchse (18) angebracht ist, die im oder auf dem proximalen Katheterende (19) befestigt ist.

## Claims

1. Device for the extraction of a spiral helix from a blood vessel with said device having a guide wire (3) which bends back on itself in its distal region where it forms into a loop structure (13) so that an anchoring part (2) is created, with the free end (7) of the guide wire (3) pointing towards the proximal end of the guide wire (3) so that it can be hooked onto the spiral helix (8) to be extracted, **characterized in that** the free end (7) of the guide wire (3) has a corkscrew-like configuration and the distal end of the loop structure (13) is of angled arrangement relative to the axis of the guide wire body.

2. The device according to claim 1, **characterized in that** the guide wire (3) has been provided with a hydrophilic coating.

3. The device according to any one of the claims 1 or 2, **characterized in that** the anchoring part (2) has the form of a hook.

4. The device according to any one of the above claims, **characterized in that** the diameter of the guide wire (3) ranges between 0.1 and 0.5 mm at its free end (7).

5. The device according to any one of the above claims, **characterized in that** the free end (7) is secured by means of a sleeve (4) on the guide wire (3) thus causing a loop (13) to form.

6. The device according to claim 5, **characterized in that** the sleeve (4) is of radiopaque design.

7. The device according to any one of the above claims, **characterized in that** the free end (7), at least partially, is of radiopaque design.

8. The device according to claim 7, **characterized in that** the free end (7) of guide wire (3) has been provided with a radiopaque coating.

9. The device according to claim 8, **characterized in that** metal dust is incorporated into the radiopaque coating.

10. The device according to any one of the above claims, **characterized in that** the free end (7) of guide wire (3) is highly flexible so that it wraps or winds around the guide wire (3) when the guide wire (3) is turned or rotated.

11. Combination comprising a device as per any one of the above claims 1 to 10 and a catheter, in particular a micro-catheter.

12. The combination according to claim 11, **characterized by** an operating system (6) in which the guide wire (3) is connected to a handle sleeve (17) in a manner so as to withstand torque and attached to a bushing (18) secured within or onto the proximal end (19) of the catheter.

## Revendications

1. Dispositif pour extraire un filament en spirale depuis un vaisseau sanguin, avec un fil de guidage **(3)** qui est recourbé sur lui-même dans sa partie distale en formant une structure en boucle **(13)** de sorte qu'il se forme une partie d'ancrage **(2),** l'extrémité libre **(7)** du fil de guidage **(3)** étant tournée en direction de l'extrémité proximale du fil de guidage **(3)** et pouvant s'accrocher au filament en spirale **(8)** à extraire, **caractérisé en ce que** l'extrémité libre **(7)** du fil de guidage **(3)** est enroulée en tire-bouchon et l'extrémité distale de la structure en boucle **(13)** est coudée par rapport à l'axe du corps du fil de guidage.

2. Dispositif selon la revendication 1, **caractérisé en ce que** le fil de guidage **(3)** présente un revêtement hydrophile.

3. Dispositif selon l'une des revendications 1 ou 2, **caractérisé en ce que** la partie d'ancrage **(2)** est conformée en crochet.

4. Dispositif selon l'une des revendications précédentes, **caractérisé en ce que** le fil de guidage **(3)** présente à son extrémité libre **(7)** un diamètre de 0,1 à 0,5 mm.

5. Dispositif selon l'une des revendications précédentes, **caractérisé en ce que** l'extrémité libre **(7)** est fixée sur le fil de guidage **(3)** avec une manchette **(4)** en formant une boucle (13).

6. Dispositif selon la revendication 5, **caractérisé en ce que** la manchette **(4)** est réalisée opaque aux rayons X.

7. Dispositif selon l'une des revendications précédentes, **caractérisé en ce que** l'extrémité libre **(7)** est réalisée au moins partiellement opaque aux rayons X.

8. Dispositif selon la revendication 7, **caractérisé en ce que** l'extrémité libre **(7)** du fil de guidage **(3)** présente un revêtement opaque aux rayons X.

9. Dispositif selon la revendication 8, **caractérisé en ce que** le revêtement opaque aux rayons X présente des inclusions de poussière métallique.

10. Dispositif selon l'une des revendications précédentes, **caractérisé en ce que** l'extrémité libre **(7)** du fil de guidage **(3)** est réalisée très souple, de sorte qu'elle s'enroule autour du fil de guidage **(3)** lors d'une rotation du fil de guidage **(3).**

11. Combinaison d'un dispositif selon l'une des revendications 1 à 10 ci-dessus avec un cathéter, en particulier un micro-cathéter.

12. Combinaison selon la revendication 11, **caractérisé par** un dispositif d'actionnement **(6)** sur lequel le fil de guidage **(3)** est relié à une douille de préhension **(17)** de façon solidaire en rotation et est placé sur une douille **(18)** qui est fixée dans ou sur l'extrémité proximale **(19)** du cathéter.
